# EUROPÄISCHE PATENTSCHRIFT

(11) **EP 3 941 829 B1**
(45) Veröffentlichungstag und Bekanntmachung des Hinweises auf die Patenterteilung: **08.07.2026**
(21) Anmeldenummer: 20716706.5
(22) Anmeldetag: 20.03.2020
(51) Int. Cl.: B64D 11/06, B60N 2/00, B60N 2/70, A61B 5/18

(54) **KISSEN FÜR EINEN FLUGZEUGSITZ MIT INTEGRIERTER SENSORELEKTRONIK**
CUSHION FOR AN AIRPLANE SEAT WITH INTEGRATED SENSOR ELECTRONICS
COUSSIN POUR UN SIÈGE D'AVION AVEC CAPTEUR INTÉGRÉ ÉLECTRONIQUE

(30) Priorität: 22.03.2019 DE 102019203979
(43) Veröffentlichungstag der Anmeldung: 26.01.2022
(73) Patentinhaber: NEVEON Austria GmbH, 4550 Kremsmünster (AT)
(72) Erfinder: HESSENBERGER, Norbert, 4694 Ohlsdorf (AT); ZIPKO, Christoph, 4600 Wels (AT); BRENNER, DI Klaus, 4209 Engerwitzdorf (AT)
(74) Vertreter: Maikowski & Ninnemann Patentanwälte Partnerschaft mbB
(86) Internationale Anmeldenummer: PCT/EP2020/057793
(87) Internationale Veröffentlichungsnummer: WO 2020/193411

(56) Entgegenhaltungen:
- WO-A1-2018/177808
- DE-A1- 102017 113 037

## Beschreibung

Die vorliegende Erfindung betrifft ein Kissen, insbesondere ein Sitzkissen für einen Flugzeugsitz mit integrierter Sensorelektronik, einen Flugzeugsitz umfassend solch ein Kissen, ein Managementsystem zur Überwachung von Parametern eines auf dem Flugzeugsitz sitzenden Passagiers und ein Verfahren zur Überwachung von Parametern eines auf dem Flugzeugsitz sitzenden Passagiers.

### Beschreibung

Flugzeughersteller und deren Zulieferer sowie Fluggesellschaften sind daran interessiert Sicherheit, Komfort und Gesundheit der Passagiere konstant zu verbessern. So können Flugzeuge mit Vorrichtungen und Anlagen zur Überwachung und Steuerung der Umweltbedingungen in der Flugzeugkabine ausgestattet sein. So kann der Passagier z.B. bestimmte Bedingungen, wie Temperatur und Luftzufuhr, oder auch die Sitzstellung, selbsttätig einstellen.

Die Elektronik in Flugzeugsitzen ist ein ständiger Treiber bei der Entwicklung neuer Innovationen im Bordbereich, insbesondere in Form von Inflight-Entertainment. Die Elektronik hat das Potenzial, viel mehr zu leisten als die Unterhaltung an Bord, wie z.B. dem Passagier zu helfen, Komplikationen wie Thrombose und andere gesundheitsbezogene Flugprobleme neben der vorausschauenden Wartung zu vermeiden.

Mit den derzeitig in Flugzeugen angewandten Systemen ist es jedoch nur begrenzt möglich, die Gesundheit, Sicherheit und den Komfort eines jeden Passagiers individuell einzuschätzen und vor potentiellen Beeinträchtigungen, wie fehlerhafte Sitzhaltung oder Beschädigungen des Sitzes rechtzeitig zu warnen. So kann die von einem Flugzeugpassagier zu Beginn des Fluges eingenommene bequeme Körperhaltung im Laufe der Flugzeit sich verändern und zu Unbehagen und gesundheitlichen Problemen führen.

So beschreibt die DE 10 2017 113 037 A1 eine Flugzeugsitzvorrichtung mit einem Sitzboden. Der Sitzboden weist eine Sitzschale als Grundkörper, auf welchem eine Polstereinheit (gebildet aus einem Schaumstoff) fest angebunden ist. Zwischen Polstereinheit und Sitzschale ist ein Sensor angeordnet, über den der Passagier ein Signal zum Ausschwenken eines Elementes zum Ablegen der Beine für den Passagier geben kann.

Ein Ansatz zur individuellen Überwachung von Sicherheit, Komfort und Gesundheit von Flugzeugpassagieren ist in der EP 3 141 482 A1 beschrieben. Hier wird ein Sitzkissen beschrieben, in welchem ein Sensor eingebaut ist, der mit dem Passagier assoziierte mechanische Vibrationen und elektrisches Rauschen detektiert. Die vom Sensor aufgenommenen Informationen werden in einem Managementsystem abgeglichen und rechtzeitige Hinweise und Warnungen an den Passagier und die Flugzeugbegleiter gegeben.

Die in der EP 3 141 482 A1 beschriebene Elektronik ist in Form z.B. eines piezoelektronischen Sensors unterhalb eines Sitzkissen oder hinter einem Rückenkissen angeordnet. Es ist auch möglich einen Sensor in ein Kopfkissen einzubauen, um den Schlafzustand eines Passagiers zu überwachen.

Nachteilig bei dem in der EP 3 141 482 A1 beschriebenen Ansatz ist jedoch die durch die Anordnung der Elektronik z.B. unterhalb des Sitzkissens bedingte geringe Messsensitivität und Messgenauigkeit.

Dies ist u.a. durch den mehrlagigen bzw. mehrschichtigen Aufbau von in Flugzeugsitzen verwendeten Sitzkissen bedingt. Derartige Sitzkissen werden aus einem Polster aus Kunststoff oder natürlichen Materialien hergestellt, wobei die Verwendung von Kunststoffschaum aus verschiedenen Gründen bevorzugt ist. So können Sitzpolster im Flugverkehr aus verschiedenen mit Flammschutzmitteln versehenen offenzelligen elastischen Weichschaumstoffen mit unterschiedlichen Raumgewichten zusammengeklebt. Anstelle der Zugabe von Flammschutzmitteln direkt in den Schaum können die Sitzkissen auch mit einem Flammschutzgewebe versehen werden, wie z.B. in der WO 2018/177808 A1 beschrieben.

Der vorliegenden Erfindung liegt nunmehr die Aufgabe zugrunde, ein Sitzkissen für einen Flugzeugsitz bereitzustellen, das eine gegenüber den bekannten Ausführungen eine verbesserte und empfindlichere Überwachung der Vitalparameter eines auf dem Sitzkissen sitzenden Passagiers sowie eine Überwachung des Wartungszustandes des Sitzkissens ermöglicht.

Diese Aufgabe wird gemäß einem ersten Aspekt mit einem Kissen mit den Merkmalen des Anspruchs 1 gelöst.

Entsprechend wird ein Kissen, insbesondere ein Sitzkissen für einen Flugzeugsitz bereitgestellt, das folgende Elemente umfasst:
- mindestens ein Polster mit einer Oberseite und einer Unterseite aus mindestens einem Kunststoffschaum;
- mindestens ein Sensorsystem, wobei das mindestens eine Sensorsystem in einem der Unterseite des Polsters benachbarten Bereich des Polsters angeordnet ist; wobei das mindestens eine Sensorsystem vom Kunstschaum des Polsters umschlossen ist,- mindestens ein zumindest eine Seite, bevorzugt die Oberseite des mindestens einen Polsters bedeckendes Flammenschutzgewebe, und
- mindestens einen Überzug auf der Oberseite des Flammenschutzgewebes,
- wobei ein weiteres Sensorsystem in dem Polster, auf dem Polster oder auf dem Flammenschutzgewebe bedeckt von dem mindestens einen Überzug oder in dem mindestens einem Überzug vorgesehen ist.

Es wird somit ein Kissen, insbesondere ein Sitzkissen oder auch ein Rückenkissen, für einen Flugzeugsitz bereitgestellt, in welchem die Sensorelektronik im Polster vorgesehen ist und somit in das Sitzkissen integriert ist; d.h. die Sensorelektronik ist Bestandteil des Sitzkissens und kann als Teil des Sitzkissens transportiert werden. Das Sensorsystem ist vom Kunststoffschaum des Polsters umschlossen. Das Sensorsystem kann z.B. in den Kunststoffschaum eingeschäumt sein oder in den Kunststoffschaum eingebettet oder (z.B. zwischen zwei Lagen) eingeklebt sein. Durch das Einbringen in das Polster wird der Sensor optimal vor Umwelteinflüssen und vorzeitiger Ablösung geschützt.

Unter der Oberseite des Polsters ist vorliegend die Seite des Polsters zu verstehen, die dem Fluggast zugewandt ist, während die Unterseite des Polsters vom Fluggast abgewandt ist und somit der mechanischen Belastung durch einen Fluggast in einem geringerem Umfang ausgesetzt ist als die Oberseite.

Das Sensorsystem ist vorliegend in einem unteren Bereich des Polsters angeordnet, insbesondere im unteren Drittel des Polsters (in Bezug auf die Ausdehnung zwischen Oberseite und Unterseite des Polsters), d.h. in dem unteren Bereich des Polsters, der vom Fluggast abgewandt ist. Diese Anordnung des Sensorsystems im unteren Bereich (oder auch an der Unterseite) des Polsters führt zu eine höheren Lebensdauer des Sensorsystems.

Erfindungsgemäß ist vorgesehen, dass mehr als ein Sensorsystem im Kissen vorgesehen ist. So kann ein zweites Sensorsystem im Polster des Kissens, auf dem Polster des Kissens oder auf dem Flammenschutzgewebe (ggfs. bedeckt von einem Überzug) vorgesehen ist. So kann zusätzlich zu dem im unteren Bereich des Polsters vorgesehenen Sensorsystems mindestens ein zweites Sensorsystem (z.B. in Form einer Sensorfolie) im oberen Bereich (d.h. im oberen Drittel) des Polsters oder auch auf der Oberseite des Polsters des Kissens angeordnet sein. Die Verwendung eines zweiten Sensorsystems bewirkt eine verbesserte Messgenauigkeit.

Wie erläutert, ist erfindungsgemäß vorgesehen, dass weitere Sensorelektronik in einem das Kissen abdeckenden Überzug integriert bzw. eingebaut ist.

Das in dem Polster des vorliegenden Kissens verwendete Sensorsystem umfasst bevorzugt Sensoren zur Druckmessung, Dehnungsmessung, Kraftmessung und/oder Durchbiegungsmessung.

Der Drucksensor stellt Daten für die (Ab)nutzung des Kissens bereit. Ein Dehnungssensor misst die Rückstellkräfte des Schaumes, wodurch Rückschlüsse auf Stabilität und Festigkeit des Schaumes gezogen werden können. Bei Druckeinwirkung auf die Sensoren wird ein elektrischer Stromfluss erzeugt, der den Grad und Dauer der Druckeinwirkung reflektiert bzw. widerspiegelt. Die Sensoreinheit ist mit einer elektronischen Steuerungseinheit mit Empfänger- und Prozessoreinheit zum Empfangen und Verarbeiten des erzeugten Stromflusses verbunden.

Es ist hervorzuheben, dass unter dem vorliegenden Sensorsystem kein Heizungssystem zu verstehen ist; d.h. die Verwendung eines Heizungssystems ist im vorliegenden Kissen ausgeschlossen.

Das vorliegend in das Kissen integrierte Sensorsystem (z.B. in Form von gedruckter Elektronik) ermöglicht vorrausschauende Wartungs- und Sensorfunktionen, wobei die mit dem Sensorsystem gewonnenen Informationen insbesondere den Flugzeugherstellern, Sitzherstellern, Fluglinien und Instandhaltungs- und Wartungsfirmen aber auch den Passagieren bereitgestellt werden.

Die gedruckte Drucksensoranordnung kann die Belegung eines Sitzes bestimmen, den Zustand des Kissens überwachen, und auch dem Fluggast parallele Vorschläge zur Vermeidung von Problemen wie Thrombosen über drahtlose Kommunikationsverfahren unterbreiten. Durch die Bestimmung der Sitzbelegung können z.B. der Einstieg und die Gewichtsverteilung im Flugzeug optimiert werden.

Im Rahmen einer vorausschauenden Wartung des Kissens (Operatoren USP) ist es möglich, über die gesamte Lebensdauer des Kissens Gewichte und Nutzungsarten aufgrund der unterschiedlichen, das Kissen benutzenden Personen zu ermitteln. Dies ermöglicht wiederum die Durchführung einer vorausschauenden Wartung, um die Kissen rechtzeitig zu wechseln, bevor die Kissen durch Überbeanspruchung oder Vandalismus an Komfort für den Fluggast verlieren. Darüber hinaus ist eine vollständige Lebenszyklusanalyse des Kissens möglich, angefangen bei den verwendeten Materialien über die Zertifizierung bis hin zur Nutzung und schließlich Informationen für das Sitzrecycling oder den Weiterverkauf an Low-Cost-Flugzeugträger. Durch die implementierte Datenspeicherung kann das Produkt während der gesamten Lebensdauer identifiziert werden und ist zudem fälschungssicher.

Des Weiteren kann das in das Kissen integrierte Sensorsystem ergonomische Vorteile für den Fluggast (Passenger USP) bieten. So kann das Sensorsystem des Kissens mit dem Fluggast über eine mobile App verbunden sein. Die mobile App liest die Daten aus dem Sensorsystem (Drucksensorarray; Dehnungssensorarray) in dem Kissen aus und kann dem Fluggast Empfehlungen für einen optimalen gesunden Sitz geben. Die Drucksensoranordnung des Sensorsystems liefert auch Informationen über die Druckverteilung und bestimmt daraus das Sitzverhalten des Fluggastes. Dies kann ein gesundes Sitzverhalten fördern, und chronische Erkrankungen, die durch schlechtes Sitzverhalten wie z.B. Thrombose verursacht wird, verhindern oder reduzieren. Darüber hinaus kann das Drucksensorsystem des Sensorsystems verwendet werden, um den Stresspegel der Fluggäste anhand ihrer Bewegungen zu erfassen.

Mit dem vorliegenden Kissen können dem Kunden (wie Flugzeughersteller, Sitzhersteller, Fluglinien und Instandhaltungs- und Wartungsfirmen, Passagier) demnach Informationen zum Zustand des Kissens als auch zum Passagier bereitgestellt werden. In Hinblick auf den Service kann unter anderem ermittelt werden, wie stark das Kissen während seiner Lebensdauer belastet wurde und wie sich das auf Härte und Abmessungen des Kissens ausgewirkt hat, und ob das Kissen beim nächsten Check im Hangar oder bei schwerwiegenden Fehlern auch zwischen einem Start bzw. einer Landung (z.B. am Gate eines Flugzeuges) ausgetauscht werden muss oder es noch weiterfliegen kann. Die in das Kissen eingebaute Elektronik meldet sich selbständig beim Servicepersonal, kennt seine Nummer und liefert alle notwendigen Informationen, um bestellt werden zu können. In Hinblick auf den Passagier kann das Sitzverhalten des Passagiers aufgezeichnet werden und bei Wunsch mittels mobiler App ergonomische Tipps an den Passagier übermittelt werden, wie dieser z.B. gesünder oder aktiver sitzen kann. Es können auch Spiele am Board-Entertainment System angeboten werden in denen die Spielfigur durch Sitzverlagerung gesteuert werden kann, wie z.B. Schifahren, Racing, Balance- oder Geschicklichkeitsspiele etc.

In einer Ausführungsform des vorliegenden Kissens umfasst das mindestens eine Sensorsystem ein flexibles Substrat mit mindestens einem auf dem flexiblen Substrat angeordneten (aufgedruckten) Sensor.

Das Sensorsystem in Form einer gedruckten Elektronik kann verschiedene Sensoren umfassen, wie z.B. RFID-Sensoren, Kontakt-freie Sensoren, piezoelektronische Sensoren. Auch können die Sensoren in Form von Messdrähten oder gewirkten, gestrickten oder gewebten Fäden vorliegen, die mäanderförmig oder jeweils horizontal und vertikal zueinander angeordnet sind. Des Weiteren kann das Sensorsystem Antennen umfassen.

In einer Ausführungsform wird ein RFID-Tag in Form eines integrierten RFID-Etiketts bereitgestellt, welches für Anwendungen auf nichtmetallischen Oberflächen geeignet ist. Dieser RFID-Tag ermöglicht eine maximale Lesereichweite für den Einsatz mit mobilen Handscannern, als auch NFC (near field communication), Bluetooth, Ultra-Low-Power Bluetooth, Comprehensive Bluetooth, Cypress Low-Power Bluetooth MCU for Mesh Networking, Bluetooth 5.2. (im Falle einer 5G Bandbreite) oder Wlan. Für Bluetooth 5.0 ICs wird die Funktion der Langstreckenkommunikation empfohlen, um die Anzahl der benötigten Lesegeräte zu reduzieren (LE-Coded Support (125 kbps)). Bluetooth 5.1 kann verwendet werden, da es als zusätzliche Funktion eine präzise Ortungs-/Richtungserkennung bietet. Im Falle einer HF-Kommunikation wären aufgrund möglicher Signalinterferenzen und kurzer Kommunikationsreichweite (~7-10m) mehrere Lesegeräte in der Ebene zu installieren.

Als flexibles Substrat, auf dem die Sensoren angeordnet sind, wird bevorzugt ein Material bzw. eine Materiallage aus Polyethylenterephthalat (PET), Polyimid (PI) oder thermoplastisches Polyurethan (TPU) verwendet. Das für das Sensorsystem verwendete Material ist flammenstabil ausgebildet.

Besonders bevorzugt ist die Verwendung von piezoresistiven, polymerbasierten Sensoren, die eine hohe Sensitivität aufweisen. Das Trägermaterial basiert auf Epoxidharzen. Die Anwendung kann als Einzelsensor oder als RFID-Sensoreinheit erfolgen.

Wie bereits erwähnt, liegt das Sensorsystem bevorzugt als gedruckte Elektronik vor. Im Falle eines gedruckten kapazitiven Sensors wird die Kapazität zwischen zwei gedruckten Elektrodenplatten (jeweils angeordnet im unteren und oberen Bereich des Polsterschaums) bestimmt, die bei einer Benutzung des Kissens aufgrund der Kompression des Schaums sich verringert. Ein gedruckter Widerstandssensor misst die Abnahme des Widerstandes der Sensorelektroden bei Benutzung des Kissens aufgrund der Kompression der Sensortinte. Ein gedruckter Dehnungssensor bestimmt die Änderung des Widerstandes eines gedruckten Dehnungsmessstreifens (der bevorzugt eingeschäumt ist, zwischen Schaumlagen eingelegt oder am Schaum aufgeklebt ist) bei Benutzung des Kissens aufgrund der mechanischen Deformierung des Dehnungsmessstreifens.

Als Drucktinten können silberhaltige Tinten oder kohlenstoffhaltige Tinte verwendet werden. In einer Ausführungsform wird ein unter Verwendung von Silbertinte auf ein Polymermaterial (z.B. PET oder TPU) gedrucktes Sensorsystem eingesetzt. Die gedruckten Sensoren können unterschiedliche Formen und Größen aufweisen. Auch kann die Sensitivität der Sensoren durch die Art, Menge und Dicke der Tinte, Variationen der Elektrodenkonfiguration eingestellt werden.

In einer besonders bevorzugten Ausführungsform wird ein gedruckter Widerstandssensor (FSR) verwendet. Solch ein Sensor besteht aus einer gedruckten Elektrode mit einer gedruckten Schicht aus einem drucksensitiven Material (z.B. Tinte). In einer Variante umfasst der FSR-Sensor eine auf einer PET-Folie oder TPU-Folie gedruckte Silberelektrode mit darauf angeordneten Isolatormaterial und drucksensitiven Material (gedruckte FSR Sensortinte), so dass bei Anwendung eines Druckes auf den Sensor ein Schalter geschlossen wird und der reduzierte Widerstand gemessen wird (z.B. durch ein MCU verbunden mit dem Sensor). Basierend auf dem Widerstand des geschlossenen Schalters kann die wirkende Kraft, Druck oder Gewicht bestimmt werden.

Zur Gewährleistung einer zuverlässigen Stromversorgung kann ein Energiespeicher (Batterie/Super-Cap) verwendet werden. Eine batteriebetriebene Lösung ist hierbei am kosten- und zeitgünstigsten.

Wie oben erwähnt, ist das Sensorsystem erfindungsgemäß vom Kunststoffschaum des Polsters umschlossen bzw. ist in den Kunststoffschaum eingebettet. So kann das Sensorsystem während der Fertigung in das Polster (oder eine andere Struktur) eingebracht werden, z.B. eingeschäumt werden. Auch ist ein integrales Einbringen zwischen Laminatlagen oder Schaumlagen möglich. Dies ist aufgrund der chemischen Kompatibilität des Sensors mit dem Material des Polsters (oder einer anderen Struktur) möglich, wobei eine optimale chemische Verbindung entstehen kann.

Das im dem vorliegenden Kissen verwendete Polster besteht aus einem Kunststoffschaum, bevorzugt aus Polyurethanschaum, Polyurethan-Graphit-Schaum Polyethylenschaum, Polystyrolschaum, Polycarbonatschaum, Polyetherschaum, Polyesterschaum, PVC-Schaum, Silikonschaum, oder PMMA (Polymethylmethacrylat)-Schaum. Bevorzugt ist die Verwendung eines Polsters aus einem (offenzelligen) Polyurethanschaum. Es ist auch möglich, dass in den Kunststoffschaum weitere Fasermaterialien, insbesondere hochtemperaturbeständige Fasern, eingebettet sind.

Polyurethanschäume werden typischerweise aus einem Isocyanat oder oder einem Gemisch von mindestens zwei Isocyanaten, wie zum Beispiel Toluol-2,4-Di isocyanat (TDI) oder Diphenylmethandiisocyanat (MDI), gegebenenfalls eingesetzt als Präpolymer, und einem Polyol oder einem Gemisch von mehreren Polyolen, beispielsweise einem Polyetherpolyol oder einem Polyesterpolyol, sowie Wasser als Basiskomponenten hergestellt. Das zumindest eine Isocyanat und dass zumindest eine Polyol werden vorzugsweise einem Anteil zugesetzt, so dass ein Verhältnis der funktionellen Gruppen -NCO/-OH in einem Bereich zwischen 0,20:1, insbesondere 0,85:1 und 0,95:1, insbesondere 1,2:1 eingestellt wird.

Es können weitere Zusatzstoffe, wie beispielsweise ein Katalysator bzw. Aktivator, Entschäumer, Farbmittel zugesetzt werden. Als Zusatzstoffe können Farbpigmente, wie Titanoxide, insbesondere Titandioxid, Bariumsulfat, Ionenaustauscher, Polyethylen, Polypropylen, Polyester, Aktivkohle, Polymere Superabsorber zur Aufnahme von Feuchtigkeit und Flammschutzmittel verwendet werden.

Generell ist zu beachten, dass die verwendeten Schäume den spezifischen Anforderungen in der Flugzeugindustrie genügen müssen. Eine einfache Übertragung von Eigenschaften von einem Schaum auf einen anderen Schaum ist in der Flugzeugindustrie nicht möglich. Insbesondere muss den strengen Anforderungen an das Brandverhalten von Schäumen in der Flugzeugindustrie entsprochen werden.

Das Polster kann eine oder mehrere Schaumschichten oder Schaumlagen umfassen. Im Falle eines mehrlagigen Polsters können die verschiedenen Schaumschichten über verschiedene Festigkeiten bzw. Steifigkeiten verfügen, die während der Schaumherstellung einstellbar sind.

Wie bereits oben angedeutet, kann das mindestens eine Sensorsystem zwischen diesen Schaumlagen eingelegt bzw. angeordnet sein. Im Falle von zwei Sensorsystemen könne z.B. folgende Schichtaufbauten des Polsters vorgesehen sein (von unten nach oben gesehen): erste Schaumlage - erstes Sensorsystem - zweite Schaumlage - opt. dritte Schaumlage - zweites Sensorsystem - opt. Flammenschutzgewebe. Insgesamt sind verschiedene Anordnungen vorstellbar, die weiter unten detaillierter beschrieben werden.

Wie oben angedeutet, wäre es auch möglich, das Sensorsystem auf der Unterseite des Polsters (z.B. mittels einer unlösbaren Verbindung) anzubringen. Eine unlösbare Verbindung kann z.B. durch Verkleben bzw. Aufkleben des Sensorsystems unter Verwendung geeigneter Klebemittel mit oder auf dem Polster erfolgen. Geeignete Klebemittel können flammgeprüfte Kleber, wie z.B. thermoplastische Co-Polyamidkleber oder wasserbasierte Kleber, sein. Besonders bevorzugt sind feuchtigkeitsreaktive Schmelzklebstoffe auf der Basis von Polyurethan-Prepolymeren, die mit der Feuchtigkeit der Umgebung unter Bildung von hochmolekularen Polymeren reagieren.

In einer weiteren Ausführungsform des vorliegenden Kissens sind die Sensoren des mindestens einen Sensorsystems im Polster im Gesäßbereich und/oder entlang der Oberschenkel eines sitzenden Passagiers angeordnet. Durch diese Anordnung der Sensoren ist eine spezifische Überwachung der während des Sitzvorganges des Fluggastes am stärksten belasteten Regionen bzw. Bereiche im Kissen möglich. Wie oben erwähnt, können hierzu auch zwei Sensorsysteme zum Einsatz kommen, wobei ein erstes Sensorsystem in unteren Drittel des Polsters und ein zweites Sensorsystem im oberen Drittel des Polsters des Kissen angeordnet sind.

Wie angeführt, ist das mindestens eine Polster mit dem Sensorsystem von mindestens einem Flammenschutzgewebe aus einem Vlies bzw. Gewebe bedeckt. Das Flammenschutzgewebe kann auf einer Seite des Polsters, d.h. auf der Oberseite oder der Unterseite, vorgesehen sein als auch das gesamte Polster (d.h. rundum von allen Seiten) abdecken.

Das vorliegend verwendete abriebfeste Flammschutzgewebe umfasst mindestens eine Barriereschicht aus mindestens einem Faservlies aus mindestens einer flammenfesten Faserart, und mindestens eine auf der Barriereschicht vorgesehene abriebfesten Schicht (Abrasionsschicht) aus mindestens einem Textilstoff mit hoher Abriebbeständigkeit aus mindestens einer Faserart, bevorzugt mindestens zwei Faserarten.

Die mindestens eine im vorliegenden Flammenschutzgewebe verwendete Abrasionsschicht weist eine Abriebbeständigkeit von mindestens 30.000 Zyklen, bevorzugt von mindesten 45.000 Zyklen, insbesondere von mindestens 55.000 Zyklen, ganz besonders bevorzugt von mindestens 150.000 Zyklen auf. Die Abriebbeständigkeit wird nach Martinedale DIN ISO 12947 1;BS 5960; 1988 bestimmt. In dem Testverfahren nach Martindale wird als Standardmaterial Baumwolle verwendet. Die erforderliche Höhe der Abriebfestigkeit des Flammenschutzgewebes ist von der anvisierten Verwendung beeinflusst. So hängt die Abriebfestigkeit von der Flugzeugstruktur ab z.B. Sitzbodenschale bzw. Gewebe (Diaframe) oder Rückenlehne Struktur, in oder an welcher das Flammenschutzgewebe eingesetzt wird. Vorliegend wird angestrebt, ein Flammenschutzgewebe bereitzustellen, welches für alle Flugzeugstrukturen einsetzbar ist.

Die hohe Abriebbeständigkeit des für die Abrasionsschicht verwendeten Textilstoffes wird insbesondere durch eine große Engmaschigkeit und geringen Faserabstand der im Textilstoff verwendeten Fasern bedingt. Entsprechend liegt die Zugfestigkeit des für die Abrasionsschicht verwendeten Textilstoffes in einem Bereich zwischen 700 und 1200 N/25mm, bevorzugt zwischen 800 und 1100 N/25mm, insbesondere bevorzugt zwischen 850 und 1000 N/25mm in der Längsrichtung und in einem Bereich zwischen 400 und 800 N /25mm, bevorzugt zwischen 500 und 700 N/25mm, insbesondere bevorzugt zwischen 500 und 600 N/25mm in der Querrichtung. Die Zugfestigkeit wird nach DIN 53357-A mit einem 25 mm breiten Band bestimmt.

Der als Abrasionsschicht verwendete Textilstoff kann ein Gewebe, ein Gewirke oder ein Gestricke sein. Dabei ist es bevorzugt, wenn der verwendete Textilstoff auf der Vorderseite und der Rückseite die gleiche Optik aufweist.

Vorliegend ist unter einem Gewebe ein textiles Flächengewebe aus mindestens zwei rechtwinklig oder nahezu rechtwinklig verkreuzten Fadensystemen zu verstehen, wobei die in Längsrichtung verlaufenden Fäden als Kettfäden bezeichnet werden und die in Querrichtung verlaufenden Fäden als Schussfäden bezeichnet werden. Die Fäden gehen in einem bestimmten Rhythmus (Bindung) über und unter den querliegenden Fäden durch. Im vorliegend als Abrasionsschicht vewendeten Gewebe liegen eine Faser in Kettrichtung und eine Faser in Schussrichtung aneinander. Hierdurch wird eine hohe Engmaschigkeit bewirkt,

Ein Gewirke und ein Gestricke hingegen sind Maschenwaren und werden aus Fadensystemen durch Maschenbildung hergestellt, wobei eine Fadenschlinge in eine andere Fadenschlinge geschlungen wird. Im Falle eines Gestrickes wird eine Masche neben der anderen hergestellt, d.h. der Faden verläuft horizontal; während im Falle eines Gewirkes der Faden übereinander stehende Maschen bildet, d.h. der Faden verläuft senkrecht. Auch hier wird durch einen geringen Faserabstand eine hohe Engmaschigkeit bewirkt.

In einer Ausführungsform kann die in der Abrasionsschicht verwendete Faserart synthetische oder natürliche Fasern umfassen.

Als synthetische Fasern können Polymerfasern ausgewählt aus der Gruppe der Polyacrylnitrilfasern (PAN-Faser), preoxidierte PAN-Fasern, Polyaramid-Fasern, wie para-Aramid-Fasern (Kevlar) oder meta-Aramid-Fasern (Nomex), Kynol-Novoloid oder Carbon-Fasern verwendet werden. Auch flammschutzausgerüstete Fasern können verwendet werden. Bevorzugte Fasern sind para-Aramid Fasern (oder alternativ meta-Aramid-Fasern) und Polyacrylnitrilfasern (PAN-Faser). Als natürliche Fasern können flammgeschützete Samenfasern wie z.B. Baumwollfasern oder flammgeschützte Bastfasern wie z.B. Hanffasern verwendet werden.

In einer weiteren Ausführungsform des vorliegenden Flammenschutzgewebes kann die Abrasionsschicht auch aus mehr als einer Faserart bestehen. Bevorzugt sind zwei, drei oder vier verschiedene Faserarten, wobei Faserart und Faseranteile beliebig kombiniert und variiert werden können. In einer bevorzugten Ausführungsform werden lediglich zwei Faserarten verwendet, wobei poly-Aramid Fasern und Polyacrylnitrilfasern (PAN-Faser) bevorzugt sind.

So können im Falle der Verwendung von zwei Faserarten in der Abrasionsschicht die quantitativen Anteile jeweils beliebig in einem Bereich zwischen 5 und 95 Gew%, bevorzugt zwischen 10 und 90 Gew%, besonders bevorzugt zwischen 20 und 80 Gew% variieren.

In einer Variante kann die Abrasionsschicht
50 bis 90 Gew%, bevorzugt 60 bis 80 Gew%, insbesondere bevorzugt 70 Gew% einer ersten Faserart, und
10 bis 50 Gew%, bevorzugt 20 bis 40 Gew%, insbesondere bevorzugt 30 Gew% einer zweiten Faserart
umfassen.

In einer besonders bevorzugten Ausführungsform des vorliegenden Flammenschutzgewebes besteht die mindestens eine Abrasionsschicht
50 bis 90 Gew%, bevorzugt 60 bis 80 Gew%, insbesondere bevorzugt 70 Gew% Polyacrylnitril (PAN) Fasern, und
10 bis 50 Gew%, bevorzugt 20 bis 40 Gew%, insbesondere bevorzugt 30 Gew% Para-aramid Fasern.

In einer weiteren bevorzugten Ausführungsform des vorliegenden Flammenschutzgewebes weist die mindestens eine Abrasionsschicht ein Flächengewicht zwischen 100 und 180 g/m², bevorzugt zwischen 110 und 150 g/m², insbesondere bevorzugt zwischen 130 und 140 g/m² auf.

Generell ist es vorstellbar und möglich, mehr als eine Textillage als Abrasionsschicht zu verwenden, z.B. zwei, drei oder vier.

Die Dicke der Abrasionsschicht liegt in einem Bereich zwischen 0,2 und 1 mm, bevorzugt 0,4 bis 0,8 mm, insbesondere bei 0,5 mm.

Wie oben erwähnt, kann die Abrasionsschicht ein Fasergemisch aus Fasern auf Basis z.B. von Polyaramiden (insbesondere para-Aramid) und Polyacrylnitril umfassen bzw. aus diesen Fasern bestehen.

Polyaramide (aromatische Polyamide) sind Polyamide, bei denen die Amidgruppen an aromatischen Gruppen gebunden sind. Aramide zählen zu den Flüssigkristallpolymeren (FKP). Die wichtigsten Typen sind Poly(p-phenylenterephthalamid) (PPTA, Handelsnamen: Kevlar, Twaron) und Poly(m-phenylenisophthalamid) (PMPI, Handelsnamen: Nomex, Teijinconex)

Polyacrylnitrilfasern (PAN-Fasern) bestehen typischerweise aus 100% Poylacrylnitril. PAN-Fasern sind hart, steif, chemikalien- und lösungsmittelbeständig und weisen einen Schmelzpunkt oberhalb der Zersetzungstemperatur auf. Es können auch Copolymer-Fasern verwendet werden, die aus Polyacrylnitril (Anteil >85 %) und Polymethylmethacrylat bestehen.

Die mindestens eine Abrasionsschicht ist bevorzugt durchgängig flächig auf die mindestens eine Barriereschicht als Teil des Flammenschutzgewebes aufgetragen, und liegt demnach bevorzugt nicht in Form eines Gitternetzes oder Gittergewebes vor.

Wie oben angeführt, besteht die mindestens eine Barriereschicht aus mindestens einem Faservlies aus mindestens einer flammenfesten Faserart.

Faservliese (oder Vliesstoffe) sind Gebilde aus Fasern begrenzter Länge, Endlosfasern (Filamenten) oder geschnittenen Garnen jeglicher Art und jeglichen Ursprungs, die auf irgendeine Weise zu einem Vlies (einer Faserschicht, einem Faserflor) zusammengefügt und auf irgendeine Weise miteinander verbunden worden sind, wobei ein Verkreuzen bzw. Verschlingen von Garnen, wie es beim Weben, Wirken, Stricken (siehe oben) geschieht, ausgenommen sind. Vliesstoffe sind größtenteils flexible textile Flächengebilde, d. h. sie sind leicht biegsam, ihre Hauptstrukturelemente sind textile Fasern und sie weisen eine vergleichsweise geringe Dicke gegenüber ihrer Länge und Breite auf.

Im vorliegenden Fall sind die Fasern des als Barriereschicht verwendeten Faservlieses vernadelt und anschließend geglättet, bzw. kalandriert (bzw. zwischen zwei Walzen verpresst oder gebügelt).

In einer Ausführungsform kann die in der Barriereschicht verwendete Faserart synthetische oder natürliche Fasern umfassen. Bevorzugt umfasst die Barriereschicht synthetische Fasern in Form vom Polymerfasern ausgewählt aus der Gruppe der Polyacrylnitrilfasern (PAN-Faser), preoxidierte PAN-Fasern, Acrylnitril-Fasern (z.B. Pyrotex), Polyaramid-Fasern, Kynol-Novoloid, para-aramid (Kevlar), meta-aramid (Nomex), Basalt-Faser, Polykieselsäure (SIALOXOL-Verbindungen), Carbon-Faser verwendet werden.

In einer weiteren Ausführungsform des vorliegenden Flammenschutzgewebes kann die Barriereschicht auch aus mehr als einer Faserart bestehen. Bevorzugt sind zwei, drei oder vier verschiedene Faserarten, wobei Faserart und Faseranteile beliebig kombiniert und variiert werden können.

So können im Falle der Verwendung von zwei Faserarten in der Barriereschicht die quantitativen Anteile jeweils beliebig in einem Bereich zwischen 5 und 95 Gew%, bevorzugt zwischen 10 und 90 Gew%, besonders bevorzugt zwischen 20 und 80 Gew% variieren.

Im Falle der Verwendung von drei Faserarten können die quantitativen Anteile wie folgt aussehen:
50 bis 90 Gew%, bevorzugt 60 bis 80 Gew%, insbesondere bevorzugt 70 Gew% einer ersten Faserart,
5 bis 30 Gew%, bevorzugt 10 bis 20 Gew%, insbesondere bevorzugt 15 Gew% einer zweiten Faserart, und
5 bis 30 Gew%, bevorzugt 10 bis 20 Gew%, insbesondere bevorzugt 15 Gew% einer dritten Faserart.

In einer besonders bevorzugten Ausführungsform des vorliegenden Flammenschutzgewebes besteht die mindestens eine Barriereschicht
50 bis 90 Gew%, bevorzugt 60 bis 80 Gew%, insbesondere bevorzugt 70 Gew% Acrylnitril-Fasern (wie Pyrotex Faser),
5 bis 30 Gew%, bevorzugt 10 bis 20 Gew%, insbesondere bevorzugt 15 Gew% Para-amid Fasern, und
5 bis 30 Gew%, bevorzugt 10 bis 20 Gew%, insbesondere bevorzugt 15 Gew% pre-oxidierte Polyacrylnitril (preox PAN) Fasern.

In einer weiteren bevorzugten Ausführungsform des vorliegenden Flammenschutzgewebes weist die mindestens eine Barriereschicht ein Flächengewicht zwischen 50 und 150 g/m², bevorzugt zwischen 60 und 120 g/m², insbesondere bevorzugt zwischen 70 und 100 g/m² auf.

Die Dicke der Barriereschicht liegt zwischen 0,7 und 1,3 mm, bevorzugt zwischen 0,9 und 1,2 mm, bevorzugt zwischen 1,0 und 1,2 mm.

Auch im Falle der Barriereschicht ist es generell vorstellbar und möglich, mehr als eine Vlieslage als Barriereschicht zu verwenden, z.B. zwei, drei oder vier.

Wie oben bereits erwähnt, kann die Barriereschicht aus einem Fasergemisch von Fasern auf der Basis von Acrylnitril-Fasern (wie Pyrotex), pre-oxidierten Polyacrylniril-Fasern und Para-Aramid Fasern bestehen.

Pyrotex-Fasern sind flammbeständige Fasern auf der Basis von Acrylnitril. Pyrotex-Fasern weisen sich durch eine hohe Säure/Basenbeständigkeit; UV Beständigkeit, Lösemittel-, Hydrolyse- und Oxidationsbeständigkeit und Dauertemperaturbeständig bis max. 250°C aus.

Pre-oxidierte Polyacrylnitril-Fasern (preox PAN Faser) sind oxidierte PAN Fasern mit einer sehr hohen Flammenbeständigkeit.

Das vorliegende Flammenschutzgewebe ist frei von Füllstoffen, wie anorganischen Füllstoffen, oder anderen Additiven. Zudem weist es keine Silikonbeschichtung oder ähnliches, wie oftmals in der Vergangenheit verwendet auf.

In einer weiteren bevorzugten Ausführungsform umfasst das vorliegende Flammenschutzgewebe mindestens eine Intumeszenz-Schicht. Im Kontext der vorliegenden Erfindung bezeichnet der Begriff der Intumeszenz eine Ausdehnung oder eine Anschwellung, also eine Volumenzunahme eines festen Körpers bzw. Materials. Intumeszente Materialien nehmen unter Hitzeeinwirkung an Volumen zu und entsprechend an Dichte ab.

Im Falle des vorliegenden Flammenschutzgewebes besteht die mindestens eine Intumeszenz-Schicht aus Blähgraphit, Kohle oder geeigneten Flammschutzfasern.

Besonders bevorzugt ist die Verwendung von Blähgraphit als Intumeszenz-Schicht. Blähgraphit, auch expandierbarer Graphit genannt, wird aus dem natürlich vorkommenden Mineral Graphit hergestellt. Eine Graphitflocke besteht aus Schichten von wabenförmig angeordneten Kohlenstoffatomen. Innerhalb der Schichten sind die Atome durch kovalente Bindungen sehr fest verbunden. Zwischen den Schichten herrschen nur schwache Bindungskräfte, so dass Moleküle zwischen die Graphitschichten eingelagert (interkaliert) werden können. Durch die Einlagerung von Säuren, üblicherweise Schwefelsäure, wird Graphit in Blähgraphit umgewandelt. Wird Blähgraphit erhitzt, expandieren die Graphitflocken je nach Qualität, ab einer Temperatur von ca. 140°C, vorliegend bei ca. 180°C auf ein Vielfaches des ursprünglichen Volumens an. Durch das Verdampfen der eingelagerten Verbindungen werden die Graphitschichten ziehharmonikaartig auseinandergetrieben. Die expandierten Flocken haben eine "würmchenartige" Erscheinungsform und sind üblicherweise mehrere Millimeter lang. Eine der Hauptanwendungen von Blähgraphit stellt der Flammenschutz dar. Bei Hitzeeinwirkung expandiert der Blähgraphit und bildet eine Intumeszenzschicht auf der Materialoberfläche. Dies verlangsamt die Brandausweitung und wirkt den für den Menschen gefährlichsten Brandfolgen, nämlich der Bildung toxischer Gase und Rauch, entgegen.

Die mindestens eine Intumeszenz-Schicht ist bevorzugt auf zwischen der Abrasionsschicht und der Barriereschicht als Zwischenlage vorgesehen.

Im Falle der Verwendung von Blähgraphit als Intumeszenz-Schicht wird das Blähgraphit zunächst in ein geeignetes Bindemittel eingebracht. Anschließend wird die Abrasionsschicht mit dieser Lösung oder Suspension beschichtet.

Die mindestens eine Blähgraphitschicht weist ein Flächengewicht zwischen 30 und 110 g/m², bevorzugt zwischen 40 und 100 g/m², bevorzugt zwischen 50 und 80 g/m² auf.

Die Dicke der Blähgraphitschicht liegt zwischen 0,1 und 0,3 mm, bevorzugt zwischen 0,1 und 0,2 mm.

In einer besonders bevorzugten Ausführungsform besteht das vorliegende Flammenschutzgewebe aus mindestens einer Abrasionschicht in Form eines Gewirkes bzw. Gewebe mit mehr als 30.000 Zyklen Abriebbeständigkeit, mindestens einer Intumeszenzschicht in Form von Blähgraphit, Kohle oder verschiedenen Flammschutzfasern, und mindestens einer Barriereschicht in Form eines Nadelfließ aus flammbeständigen Materialien.

Der Schichtaufbau des vorliegenden Flammenschutzgewebes ist in einer bevorzugten Variante (von oben nach unten gesehen): Abrasionsschicht - Intumeszenzschicht-Barriereschicht.

Die Gesamtdicke des vorliegend verwendeten Flammenschutzgewebes liegt in einem Bereich zwischen 1,5 und 2,5 mm, bevorzugt zwischen 1,8 und 2,0 mm bei einem Gesamtgewicht zwischen 200 und 300 g, bevorzugt zwischen 220 und 280 g, insbesondere bevorzugt zwischen 250 und 270 g.

In einer weiteren Variante ist vorgesehen, dass der Schichtaufbau aus Abrasionsschicht, optional Intumeszenschicht und Barriereschicht laminiert, kaschiert, verklebt oder vernadelt wird.

In einer bevorzugten Ausführungsvariante des vorliegenden Kissens ist zwischen dem mindestens einen Sensorsystem und dem mindestens einen Flammenschutzgewebe mindestens ein Schaumteil bzw. eine Schaumlage vorgesehen. Die zusätzliche Schaumlage dient insbesondere dazu, dass der Passagier die Sensorik im Kissen nicht in unangenehmer Weise spüren kann.

Die zusätzliche Schaumlage besteht bevorzugt aus Polyurethanschaum, Polyethylenschaum, Polyetherschaum, Polyesterschaum, Silikonschaum oder kann auch ein Kunststoffgewebe, bevorzugt aus Polypropylen, Polyethylen, Poylacrylat umfassen. Es ist auch möglich, in die Schaumlage Blähgraphit einzuschäumen.

Das mindestens eine Flammenschutzgewebe ist mit dem mindestens einem Schaumteil verklebt.

Erfindungsgemäß ist auf der Oberseite des Flammschutzgewebes mindestens ein Überzug vorgesehen sein. Der mindestens eine Überzug kann mit dem mindestens einen Flammenschutzgewebe laminiert, kaschiert, verklebt oder vernäht sein. Dieser Überzug kann aus Leder, Kunstleder oder einem (haptisch angenehmen) Dekorstoff hergestellt sein.

In einer bevorzugten Ausführungsform wird ein Überzug (für das Kissen oder für die Rückenlehne) aus Funktionstextilien (wie. z.B. Goretex) verwendet. Derartige Funktionstextilien werden heutzutage in sämtlichen Bereichen des Lebens verwendet, insbesondere im Sport. Diese Funktionstextilien sind je nach Einsatz winddicht, wasserdicht, atmungsaktiv, thermoregulierend, schmutzabweisend, antimikrobiell, flammhemmend, UVbeständig, elektrisch abschirmend, elastisch, strapazierfähig, pflegeleicht, chemikalienresistent, leicht, wärmend/kühlend. Vorliegend verhindert ein Überzug aus Funktionstextilien insbesondere das Rückschwitzen und die Verhinderung des Wärmestaus der Passagiere. Durch die atmungsaktiven Textilien wird der Schweiß vom Passagier abtransportiert.

Die erwähnten Materialien des Überzuges für Kissen oder Rückenlehne sind bevorzugt auf einen dünnen Schaumstoff laminiert. Die Dicke des Überzugschaustoffes kann zwischen 5 und 15 mm, bevorzugt 10 mm betragen

Erfindungsgemäß ist im Überzug (für Sitzkissen und/oder Rückenlehen) ebenfalls ein Sensorsystem vorgesehen. Das in den Überzug eingebaute Sensorsystem dient bevorzugt der Messung von Druck, Temperatur und Feuchtigkeit. Die Verwendung eines Temperatursensors im Überzug ermöglicht die Bereitstellung von Informationen bezüglich der Temperaturentwicklung im Sitz und/oder Rückenlehne und somit auch über den Zustand der Klimaanlage. Ein Feuchtigkeitssensor im Überzug kann z.B. eine Verunreinigung des Kissens durch Flüssigkeiten bestimmen.

Als Sensorsystem im Überzug werden insbesondere Fäden aus leitfähigen Materialien verwendet, die in das Gewirk, Gestrick, Gewebe eingebaut sind, oder zwischen Leder-/ Kunstledermaterial und Schaumstoff aufkaschiert sind. Da Kunstleder aus mehreren Schichten besteht, können die leitfähigen Materialien auch direkt in das Kunstleder eingearbeitet sein.

Der Überzug kann in verschiedenen Varianten hin Verbindung mit dem Kissen vorgesehen sein:
- Textil / Leder / Kunstleder mit eingearbeiteten Sensoren - Schaumlage - Kissen mit oder ohne integrierte(s) Sensorsystem(e)
- Textil / Leder / Kunstleder mit eingearbeiteten Sensoren - keine Schaumlage - Kissen mit oder ohne integrierte(s) Sensorsystem(e)

Das in den Überzug eingebaute Sensorsystem bzw. Sensorarray kann verschiedene Sitzpositionen (z.B. 5 unterschiedliche Sitzpositionen) und Sitzhaltungen erkennen. Es erlaubt eine Zeiterfassung der Sitzungsdauer, ein Vergleichen vom Sitzverhalten mit den Gesundheitsrichtlinien, ein Eingreifen und zum Wechsel der Sitzhaltung ermutigen. Die Flugbesatzung kann den Status der Sitztemperatur bei Überhitzung oder Unterkühlung erkennen und so die Passagiere bei Rückenschweiß oder Frieren pro-aktiv mit einer Wolldecke unterstützen oder den Deckenventilator einstellen.

Wie oben ausgeführt, wird das vorliegende Kissen bevorzugt für Flugzeugsitze zur Bestimmung der Sitzbelegung, Überwachung des Abnutzungsgrades und einer vorrausschauenden Wartung sowie des Sitzverhaltens des Fluggastes in Kombination mit einem computerimplementierten Verfahren verwendet.

Hierfür wird ein Managementsystem bereitgestellt, welches
- mindestens eine Sensoreinheit in einem der vorhergehend beschriebenen Kissen und/oder Überzug;
- mindestens eine Verarbeitungseinheit (processing unit, PU) zur Verarbeitung der von der Sensoreinheit gemessenen Daten;
- mindestens eine Speichereinheit (storage unit, SU) zur Speicherung der gemessenen und verarbeiteten Daten, und
- mindestens eine Anzeigeeinheit (display unit) zur Anzeige der verarbeiteten Daten umfasst.

Die mindestens eine Anzeigeeinheit kann auf einem mobilen Gerät wie z.B. einem Smartphone, Tablet oder auf stationär z.B. in der Rückenlehne eines Flugzeugsitzes vorgesehen sein. So kann das Sensorsystem des Kissens mit dem Fluggast über eine mobile App auf dem Smartphone verbunden sein. Die mobile App liest die Daten aus dem Druckarray in der Sitzschale aus und kann dem Fluggast Empfehlungen für einen optimalen gesunden Sitz geben.

Die von der Sensoreinheit gemessenen und verarbeiteten Daten werden drahtlos z.B. mittels Bluetooth (oder anderer Systeme, siehe oben) an das mindestens eine Display übertragen.

Die Verarbeitungseinheit kann auch über ein Netzwerk Interface mit dem Netzwerksystem des Flugzeuges in Verbindung stehen, und so auf mögliche Datenbanken zurückgreifen, in denen z.B. Gesundheitsdaten des Fluggastes gespeichert sind. Dies kann ein Server on Board oder in ein Cloud System (AWS-Amazon Web service, Microsoaft Azure, Google, Crossplattformen, Docker cloud hosting (Airbus Skywise) oder weitere adäquate Systeme anderer Flugzeughersteller sein.

Es ist auch vorstellbar, dass das vorliegende Managementsystem ein Managementmodul mit individuellen Daten zur Gesundheit, Sicherheit und Komfort von Passagieren aufweist.

In einer Ausführungsform umfasst das vorliegende Managementsystem folgende Komponenten: Sensoren (z. B. 8 Sensoren) als Sensoreinheit; Steuerungseinheit; Verarbeitungs- und Speichereinheit; Tablet oder Smartphone als Display. Die Sensoren werden über Bluetooth in Verbindung mit dem Tablet (oder Smartphone) entsprechend eigenständig betrieben und ermöglichen Kontakt zum Hersteller der Software und zur Ansteuerung der Sensoren.

Das beschriebene Managementsystem ermöglicht die Durchführung eines computerimplementierten Verfahrens zur Bestimmung der Sitzbelegung, Überwachung des Abnutzungsgrades und einer vorrausschauenden Wartung sowie des Sitzverhaltens des Fluggastes.

Das computerimplementierte Verfahren umfasst:
- Messen der Änderung von mindestens einem Parameter ausgewählt aus Temperatur, Druck und/oder Dehnung durch die im Kissen und/oder im Überzug angeordnete Sensoreinheit, wobei die Parameteränderung von einem auf dem Kissen sitzenden Fluggastes ausgeübt / hervorgerufen wird;
- Verarbeiten der gemessenen Daten in der mindestens einen Verarbeitungseinheit und Speichern der Daten in der mindestens einen Speichereinheit des Managementsystems, und
- Anzeigen der verarbeiteten Daten auf der mindestens einen Anzeigeeinheit.

Die Durchführung des beschriebenen computerimplementierten Verfahrens wird durch ein geeignetes Computerprogramm vorgenommen. Dieses Computerprogramm umfasst Befehle, die bei der Ausführung des Programms durch einen Computer diesen veranlassen das (computerimplementierte) Verfahren auszuführen.

Das Computerprogramm ist in der Speichereinheit des Managementsystems oder auf einem geeigneten Datenträger gespeichert. Speichereinheit des Managementsystems und Datenträger sind dabei als computerlesbares Speichermedium zu verstehen. Bevorzugt ist das Computerprogramm Teil der Plattform des Flugzeuges. Das Computerprogramm kann eigenständig online in Verbindung oder offline ohne Verbindung mit der Plattform im Flugzeug angewendet werden.

Die ermittelten Daten können dem Flugpersonal in der Kabine und/oder dem Fluggast angezeigt werden und können verschieden verwendet werden.

So kann durch die Bestimmung der Sitzbelegung z.B. der Einstieg und die Gewichtsverteilung im Flugzeug optimiert werden, wodurch eine Einsparung an Treibstoff (Kerosin) möglich ist.

Die ermittelten Daten geben auch eine Aussage über die Länge und Stärke der Nutzung des Kissens. Dies ermöglicht wiederum die Durchführung einer vorausschauenden Wartung, um die Kissen rechtzeitig zu wechseln. Auch kann mit dem vorliegenden Verfahren das Kissen aufgrund der Integration der Sensorelektronik in das Kissen während der gesamten Lebensdauer identifiziert werden.

Des Weiteren ermöglicht das vorliegende Verfahren Informationen für den Fluggast betreffend seines Sitzverhaltens bereitzustellen, die den Fluggast auf mögliche Folgen seines Sitzverhaltens hinweisen. So kann im Falle eines starken Abweichens der gemessenen Daten von einem Standardwert, eine Meldung an den Passagier und/oder auch an die Flugbegleiter übermittelt werden, um auf mögliche Probleme aufmerksam zu machen.

Die Anordnung der Sensoreinheit im Kissen ermöglicht auch die Durchführung von Studien zur Druckverteilung im Kissen bei Benutzung durch einen Passagier (Pressure mapping). So kann die Druckverteilung im Kissen Informationen über Bereiche mit hohen Druck und Haltungsabnormalitäten liefern. Basierend auf diesen Informationen kann die Stabilität und der Sitzkomfort verbessert werden, wie z.B. die Verwendung eines stabileren Schaums im Bereich der Kniekehlen zur Verbesserung der Sitzposition und Gewichtsreduzierung.

Die Erfindung wird nachfolgend unter Bezugnahme auf die Figuren der Zeichnungen näher erläutert. Es zeigen:
- Figur 1: eine schematische Ansicht eines Kissens mit integrierter Elektronik gemäß einer ersten Ausführungsform,
- Figur 2A: eine schematische Ansicht eines ersten, in ein Kissen integrierten Sensorsystems;
- Figur 2B: eine schematische Ansicht eines zweiten, in ein Kissen integriertes Sensorsystems;
- Figur 3: ein Blockdiagramm einer Ausführungsform eines Managementsystem zur Verwendung eines in ein Kissen integrierten Sensorsystems;
- Figur 4A: eine schematische Ansicht eines Kissens mit integrierter Elektronik gemäß einer zweiten Ausführungsform;
- Figur 4B: eine schematische Querschnittsansicht einer in einem Kissen der Fig. 4A integrierten Trägerschiene; und
- Figur 5A: eine schematische Ansicht eines Aufbaus eines Kissens mit einem Sensorsystem;
- Figur 5B: eine schematische Ansicht eines weiteren Aufbaus eines Kissens mit einem Sensorsystem;
- Figur 5C: eine schematische Ansicht eines Aufbaus eines Kissens mit zwei Sensorsystemen;
- Figur 5D: eine schematische Ansicht eines weiteren Aufbaus eines Kissens mit zwei Sensorsystemen;
- Figur 5E: eine schematische Ansicht eines weiteren Aufbaus eines Kissens mit zwei Sensorsystemen;
- Figur 5F: eine schematische Ansicht eines Aufbaus eines Kissens mit einem Überzug mit Sensorsystemen; und
- Figur 5G: eine schematische Ansicht eines weiteren Aufbaus eines Kissens mit einem Überzug mit Sensorsystemen.

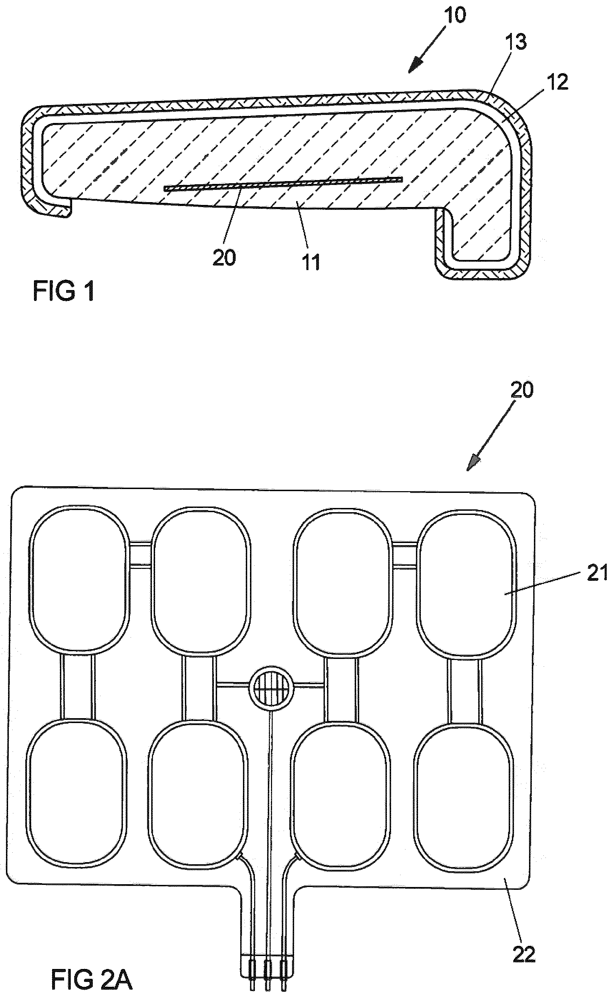

Figur 1 zeigt eine erste Ausführungsform eines Kissens 10 mit integrierter Elektronik für einen Flugzeugsitz. Der oberseitige Sitzbereich des Polsters 11 ist von einem Flammschutzgewebe 12 vollständig umgeben. Ein Schutzüberzug bzw. Abdeckung 13 ist mit dem Polster inkl. Flammschutzgewebe mittels Haft oder Flauschbänder befestigt. Der Überzug 13 erstreckt sich über die gesamte Oberseite des Polsters inklusive des Kniekehlenbereiches.

Im Polster 11 ist ein Sensorsystem 20 vorgesehen. Das im Polster 11 angeordnete Sensorsystem 20 umfasst Druck- und Dehnungssensoren, die innerhalb des Polsters 11 in einem Bereich benachbart zur Unterseite des Polsters 11 eingeschäumt sind. Durch das Einbringen des Sensorsystems in das untere Drittel des Polsters 11 wird der Sensor optimal vor Umwelteinflüssen geschützt. Es ist ebenfalls möglich, dass zusätzlich ein zweites Sensorsystem im oberen Bereich (im oberen Drittel) des Polsters vorgesehen ist (nicht gezeigt).

Das Flammenschutzgewebe 12 besteht aus einer Barriereschicht und einer Abrasionsschicht. Die Abrasionsschicht ist auf einer Seite (hier auf der Oberseite) der Barriereschicht flächig vorgesehen.

Die Barriereschicht besteht aus einem Faservlies aus 70% Pyrotex(bi-grade) Fasern (Acrylnitrilfasern), 15% para-Aramid Fasern(regeneriert) und 15% preox PAN (Polyacrylonitrile) Fasern. Das Flächengewicht der Barriereschicht beträgt in diesem Fall 70g/m². Die Abrasionsschicht besteht aus einem Gewebe aus 70% PAN (Polyacrylonitril) Fasern und 30% para-Aramid Fasern. Das Flächengewicht der Abrasionsschicht liegt bei 130 g/m².

Zusätzlich zu Barriereschicht und Abrasionsschicht kann im Flammenschutzgewebe eine dritte Intumeszenzschicht aus Blähgraphit vorgesehen sein, wobei der Blähgraphit bei 180°C beginnt aufzublähen. Die Blähgraphitschicht ist als Zwischenlage zwischen der Abrasionsschicht und der Barriereschicht vorgesehen. Die Blähgraphitschicht ist mit einem Flächengewicht von 50 g/m² auf die Abrasionsschicht aufgetragen.

Es ist auch möglich, dass das Flammenschutzgewebe und ein Schaumteil (oder Schaumlage) aus div. Schäumen miteinander verklebt oder laminiert sind. In diesem Fall ist die Sensorelektronik zwischen Polster und Schaumlage vorgesehen (nicht gezeigt).

Fig. 2A zeigt den Aufbau eines ersten in einem Kissen integrierten Sensorsystems 20. Dieses erste Sensorsystem (in Form einer RFID-Sensoreinheit) umfasst acht Sensoren 21 in Form von Polymer-Dehnungsmesssensoren. Das Trägermaterial 22 ist epoxidharzbasiert. Das Sensorsystem 20 erfasst die Längenänderung (Dehnung) bei mechanischer Verformung eines Bauteils aus Polymerverbundwerkstoffen. Der Widerstandsbereich und k-Faktor des Sensors sind anpassbar. Der Sensorsystem 20 ist auf das Polster 11 geklebt.

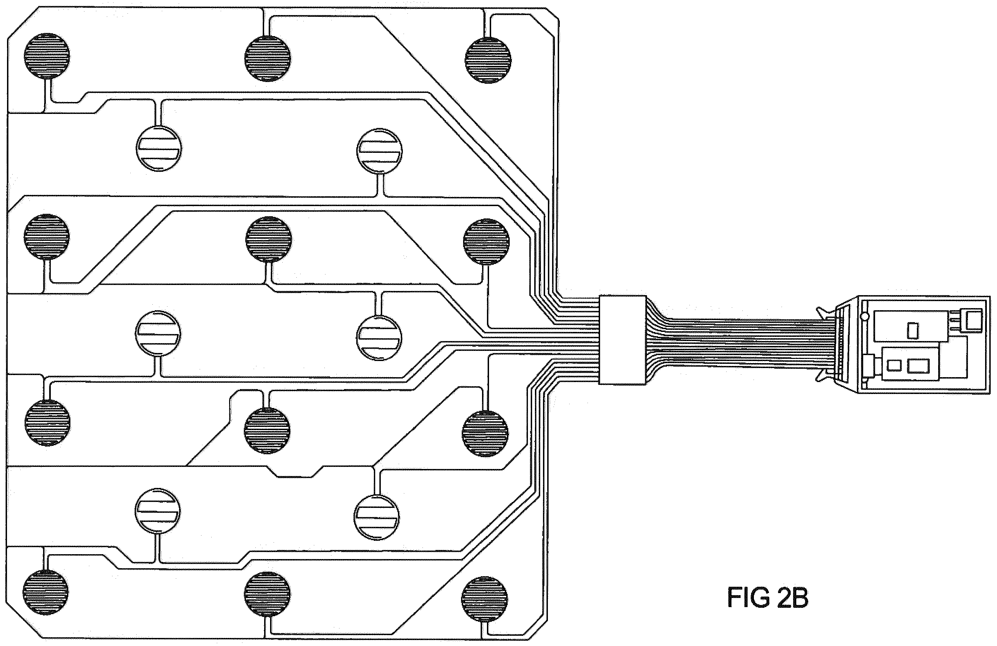

Fig. 2B zeigt den Aufbau einer zweiten Variante eines Sensorsystems 20, welches in ein Kissen integriert werden kann.

Das in Fig. 2B dargestellte Sensorsystem umfasst FSR-Drucksensoren (insgesamt 12; dunkel dargestellt) und weitere Sensoren (insgesamt 6). Die FSR-Drucksensoren umfassen jeweils eine auf einer PET-Folie oder TPU-Folie gedruckte Silberelektrode mit drauf angeordneten Isolatormaterial und drucksensitiven Material (gedruckte FSR Sensortinte), so dass bei Anwendung eines Druckes auf den Sensor ein Schalter geschlossen wird und der reduzierte Widerstand gemessen wird (z.B. durch ein MCU verbunden mit dem Sensor). Die 6 weiteren Sensoren sind zb. Sensoren die dafür sorgen dass der Sensor nur aktiv (Einsparung Energieverbrauch) ist sobald dieser belegt ist, so dass "belegt" oder "nicht belegt" angezeigt wird

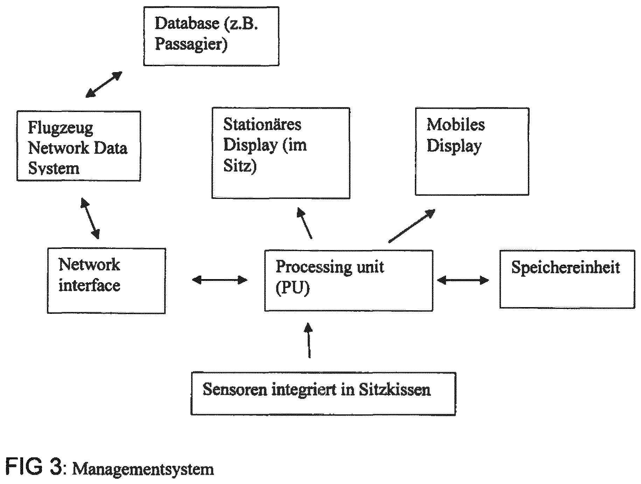

Fig. 3 zeigt ein Blockdiagramm eines Managementsystems zur Verwendung eines in ein Kissen integrierten Sensorsystems. Das Managementsystem besteht aus einer in ein Kissen integrierten Sensoreinheit; eine Verarbeitungseinheit (processing unit, PU) zur Verarbeitung der von der Sensoreinheit gemessenen Daten; eine Speichereinheit (storage unit, SU) zur Speicherung der gemessenen und verarbeiteten Daten, und eine Anzeigeeinheit (display unit) zur Anzeige der verarbeiteten Daten.

Die Anzeigeeinheit kann auf einem mobilen Gerät wie z.B. einem Smartphone und/oder Tablet oder auf stationär z.B. in der Rückenlehne eines Flugzeugsitzes vorgesehen sein. So kann das Sensorsystem des Kissens mit dem Fluggast über eine mobile App auf dem Smartphone und/oder Tablet verbunden sein. Die mobile App liest die Daten aus dem Druckarray in der Sitzschale aus und kann dem Fluggast Empfehlungen für einen optimalen gesunden Sitz geben.

Die von der Sensoreinheit gemessenen und verarbeiteten Daten werden drahtlos z.B. mittels Bluetooth an das mindestens eine Display übertragen.

Die Verarbeitungseinheit kann auch über ein Netzwerk Interface mit dem Netzwerksystem des Flugzeuges in Verbindung stehen, und so auf mögliche Datenbanken zurückgreifen, in denen z.B. Gesundheitsdaten des Fluggastes gespeichert sind.

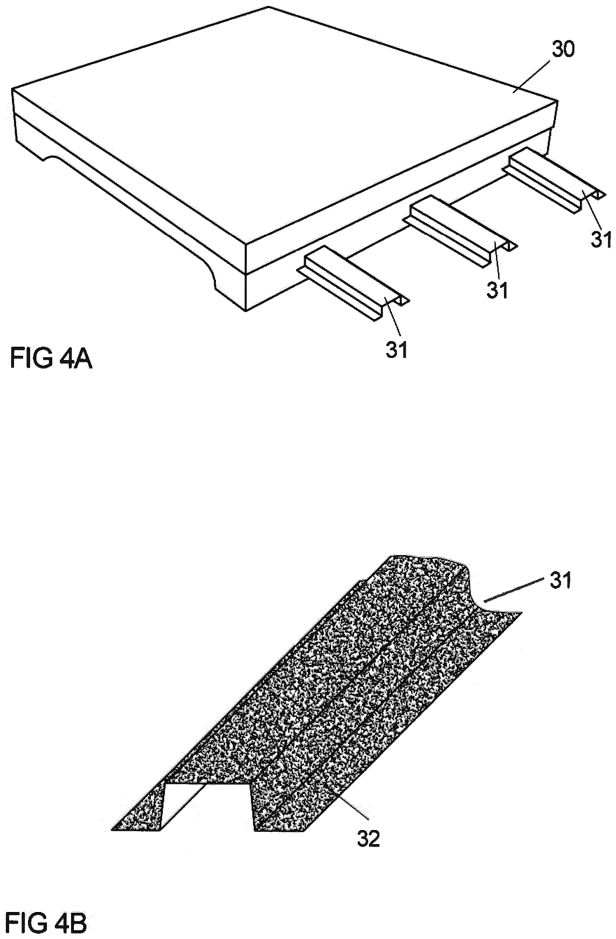

In der in den Figur 4A gezeigten Ausführungsformen des Kissens mit einem Polster 30 ist ein Stabilisierungsmittel in Form von U-förmigen Trägerschienen 31 vorgesehen.

Die Trägerschienen 31 weisen ein U-Profil auf. Die Wanddicke der Trägerschienen 31 beträgt zwischen 1,5 und 2 mm. Die Trägerschienen 31 bestehen aus Kohlenstofffasern oder Glasfasern. Die Fasern sind in ein Harzsystem eingelagert, wie z.B. in ein Phenolharz oder Epoxidharz. Die Profilierung der Trägerschienen kann z.B. durch Pressprägung von Fasernasslaminaten oder mittels dem Sheet Moldig Compound (SMC) Verfahren erfolgen.

In der in Figur 4A gezeigten Ausführungsform sind drei Trägerschienen 31 parallel mit einem Mitte-Mitte-Abstand von 100 mm in einem Polster 30 (mit einer Breite von 450 mm) gezeigt. Die Trägerschienen 31 weisen ein U-Profil oder ein Trapez-Profil auf. Die Tiefe des U-Profils bzw. Trapez-Profils liegt in einen Bereich zwischen 10 und 12 mm.

Die Trägerschienen 31 sind entweder aus einem flammfesten Material hergestellt oder sind zusätzlich mit einem Flammschutzmaterial in Form eines flammfesten Gewebes 32 aus temperaturstabilen Fasern sind Kunststofffasern auf der Basis von Polypropylen, Polyacrylat oder Polyamiden wie Aramiden oder Polybenzimidazol versehen. Hierzu können die Trägerschienen 31 mit dem flammfesten Gewebe 32 laminiert werden (siehe Figur 4B).

An den Trägerschienen ist ein Sensorsystem vorgesehen. Die in Figur 2 beschriebene Sensoreinheit kann mit dem Material der Trägerschienen verpresst, laminiert oder auf die Trägerschienen aufgeklebt sein.

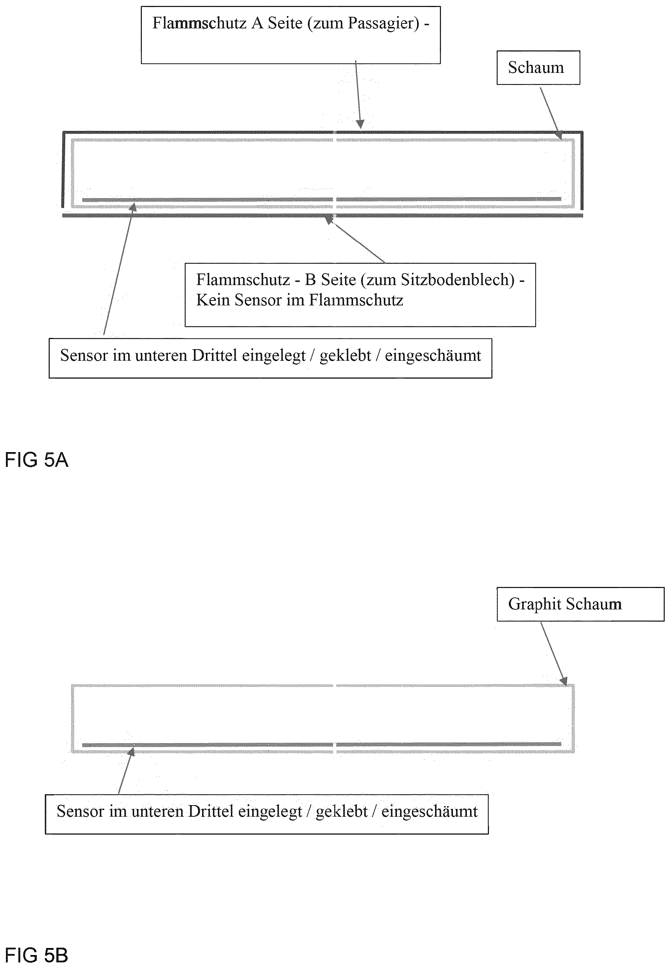

Figuren 5A-I zeigen verschiedene Varianten der Anordnung und Kombination von Kissen mit Sensorsystem und Überzug.

Figur 5A zeigt ein Kissen oder Rückenkissen in einer Anwendung mit einem rundum Flammschutz und einem im unteren Drittel des Polsters vorgesehenen Sensor. Figur 5B zeigt ein Kissen oder Rückenkissen in einer Anwendung ohne Flammschutz und einem im unteren Drittel des Polsters vorgesehenen Sensor. In einer anderen Variante ist es auch möglich, den Flammschutz lediglich auf einer Seite des Polsters, d.h. auf der Oberseite oder der Unterseite anzubringen (nicht gezeigt).

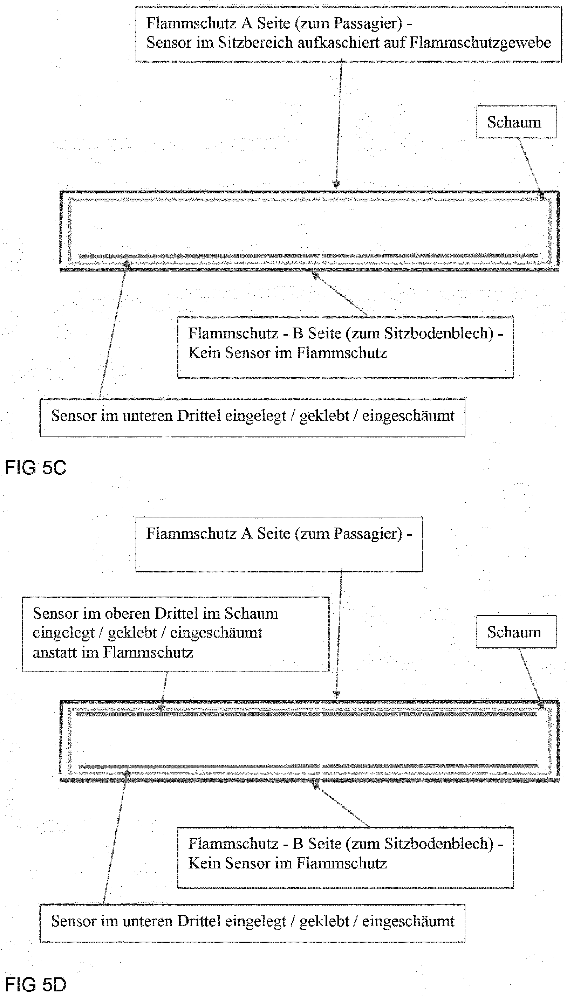

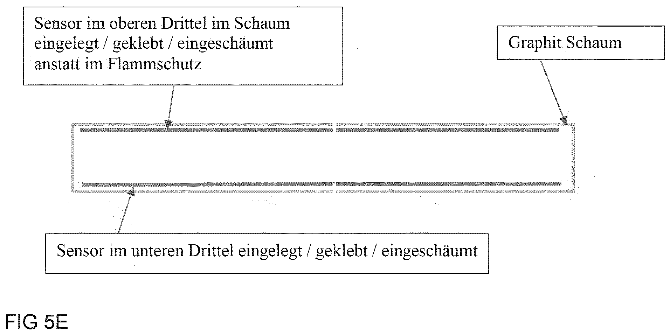

Figur 5C zeigt ein Kissen oder Rückenkissen zur Anwendung mit rundum Flammschutz und 2 Sensoren, wobei ein erster Sensor im unteren Drittel des Polsters vorgesehen ist und ein zweiter Sensor auf das Flammschutzgewebe aufkaschiert ist. Im Kissen der Figur 5D ist im Unterschied zum Kissen der Figur 5C der zweite Sensor im oberen Drittel des Polsters vorgesehen. Figur 5E zeigt das Kissen der Figur 5D ohne Flammschutzgewebe.

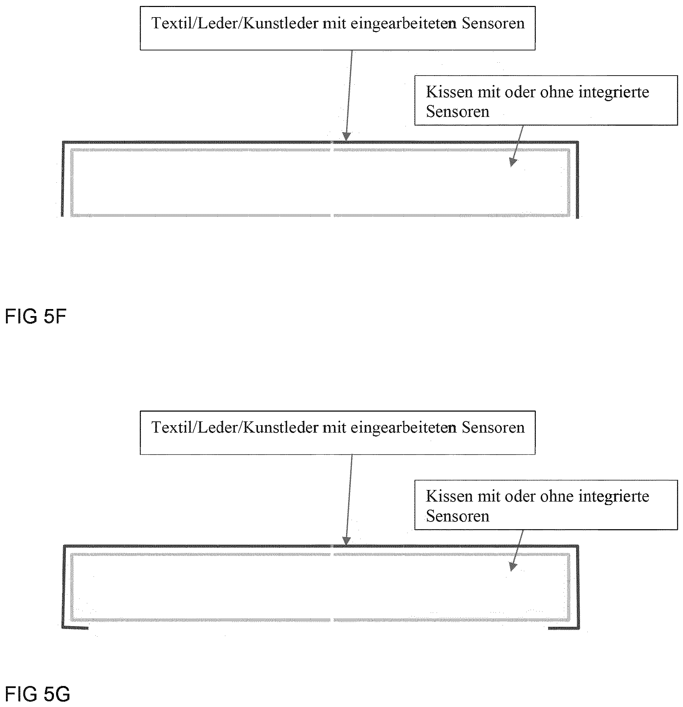

Figur 5F zeigt ein Kissen mit einem teilweisen Überzug, in welchen Sensoren eingearbeitet sind, und zwischen Polster (mit oder ohne Sensoren) und Überzug eine Schaumlage vorgesehen ist. In der Variante der Figur 5G erstreckt sich der Überzug bis auf die Unterseite des Polsters,

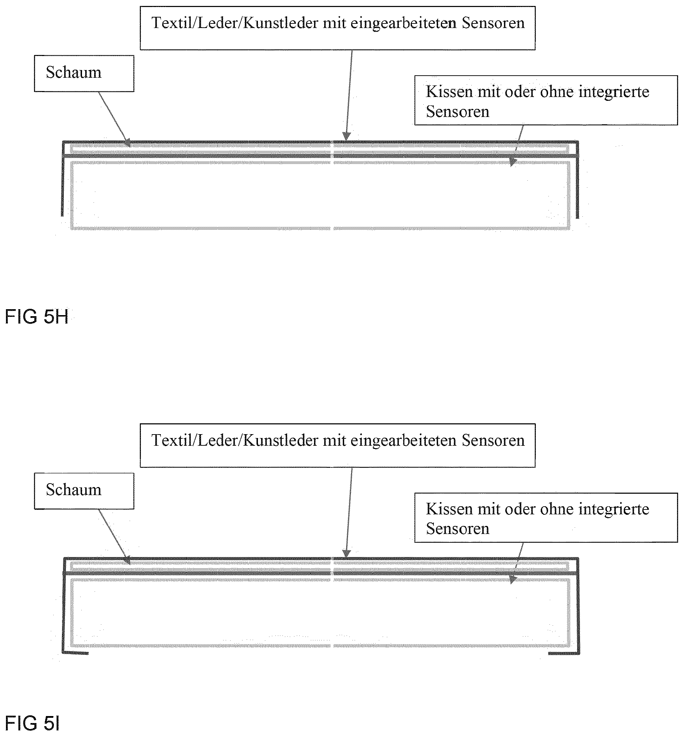

Figur 5H zeigt ein Kissen mit einem teilweisen Überzug, in welchen Sensoren eingearbeitet sind, und zwischen Polster (mit oder ohne Sensoren) und Überzug keine Schaumlage vorgesehen ist. In der Variante der Figur 5I erstreckt sich der Überzug bis auf die Unterseite des Polsters,

Die in den Figuren 5f-I gezeigten Varianten, in welche keine Sensoren im Polster vorgesehen sind, sind nicht erfindungsgemäß.

## Patentansprüche

1. Sitzkissen (10) für einen Flugzeugsitz, umfassend
- mindestens ein Polster (11) mit einer Oberseite und einer Unterseite aus mindestens einem Kunststoffschaum;
- mindestens ein Sensorsystem (20), wobei das mindestens eine Sensorsystem in einem der Unterseite des Polsters benachbarten Bereich des Polsters angeordnet ist, **dadurch gekennzeichnet dass** das mindestens eine Sensorsystem vom Kunstschaum des Polsters umschlossen ist, wobei das Sitzkissen weiter umfasst:
- mindestens ein zumindest eine Seite, bevorzugt die Oberseite des mindestens einen Polsters bedeckendes Flammenschutzgewebe (12),und
- mindestens einen Überzug (13) auf der Oberseite des Flammenschutzgewebes (12),
- wobei ein weiteres Sensorsystem in dem Polster (11), auf dem Polster (11) oder auf dem Flammenschutzgewebe (12) bedeckt von dem mindestens einen Überzug (13) oder in dem mindestens einem Überzug (13) vorgesehen ist.

2. Sitzkissen nach Anspruch 1 , **dadurch gekennzeichnet, dass** das mindestens eine im Polster vorgesehene Sensorsystem Sensoren zur Druckmessung und/oder Dehnungsmessung umfasst.

3. Sitzissen nach Anspruch 1, **dadurch gekennzeichnet, dass** das im Überzug vorgesehene Sensorsystem der Messung von Temperatur und/oder Feuchtigkeit dient.

4. Sitzkissen nach einem der vorhergehenden Ansprüche, **dadurch gekennzeichnet, dass** das mindestens eine Sensorsystem ein flexibles Substrat mit mindestens einem auf dem flexiblen Substrat angeordneten Sensor umfasst.

5. Sitzkissen nach einem der vorhergehenden Ansprüche, **dadurch gekennzeichnet, dass** das mindestens eine Sensorsystem in den Kunststoffschaum des Polsters eingeschäumt oder eingebettet ist.

6. Sitzkissen nach einem der vorhergehenden Ansprüche, **dadurch gekennzeichnet, dass** die Sensoren des mindestens einen Sensorsystems in dem Polster im Gesäßbereich und/oder entlang der Oberschenkel eines sitzenden Passagiers angeordnet sind.

7. Sitzkissen nach einem der vorhergehenden Ansprüche, **gekennzeichnet durch** mindestens ein Flammschutzgewebe umfassend
- mindestens eine Barriereschicht umfassend mindestens ein Faservlies aus mindestens einer flammenfesten Faserart, und
- mindestens einer auf der Barriereschicht vorgesehene abriebfesten Schicht umfassend mindestens einen Textilstoff mit hoher Abriebbeständigkeit aus mindestens einer Faserart, bevorzugt mindestens zwei Faserarten.

8. Sitzkissen nach einem der vorhergehenden Ansprüche, **dadurch gekennzeichnet, dass** das mindestens eine Flammenschutzgewebe mindesten eine Intumeszenz-Schicht aufweist.

9. Sitzkissen nach einem der vorhergehenden Ansprüche, **dadurch gekennzeichnet, dass** das mindestens eine Polster aus einem Kunststoffschaum, bevorzugt aus Polyurethanschaum, Polyethylenschaum, Polyetherschaum, Polyesterschaum, Silikonschaum oder aus einem Kunststoffgewebe, bevorzugt aus Polypropylen, Polyethylen, Polyacrylat besteht.

10. Flugzeugsitz, umfassend mindestens ein Sitzkissen nach einem der vorhergehenden Ansprüche.

11. Verwendung von Daten gemessen von Sensorsystemen angeordnet in einem Sitzkissen nach einem der Ansprüche 1-11 in Kombination mit einem Managementsystem zur Überwachung des Abnutzungsgrades und einer vorrausschauenden Wartung des Sitzkissens sowie des Sitzverhaltens des Fluggastes, wobei das Managementsystem
- mindestens eine Verarbeitungseinheit (processing unit, PU) zur Verarbeitung der von den im Sitzkissen angeordneten Sensorsystemen gemessenen Daten;
- mindestens eine Speichereinheit (storage unit, SU) zur Speicherung der gemessenen und verarbeiteten Daten, und
- mindestens eine Anzeigeeinheit (display unit) zur Anzeige der verarbeiteten Daten umfasst.

12. Verwendung von Daten gemessen von Sensorsystemen angeordnet in einem Sitzkissen nach einem der Ansprüche 1-11 in einem computerimplementierten Verfahren zur Bestimmung der Sitzbelegung, Überwachung des Abnutzungsgrades und einer vorrausschauenden Wartung des Sitzkissens sowie des Sitzverhaltens des Fluggastes wobei das computerimplementierte Verfahren die Schritte umfasst:
- Messen der Änderung von mindestens einem Parameter ausgewählt aus Temperatur, Druck und/oder Dehnung durch die im Kissen nach einem der Ansprüche 1-11 angeordneten Sensorsysteme, wobei die Parameteränderung von einem auf dem Sitzkissen sitzenden Fluggastes ausgeübt / hervorgerufen wird;
- Verarbeiten der gemessenen Daten in der mindestens einen Verarbeitungseinheit und Speichern der Daten in der mindestens einen Speichereinheit des Managementsystems, und
- Anzeigen der verarbeiteten Daten auf der mindestens einen Anzeigeeinheit.

## Claims

1. A seat cushion (10) for an airplane seat, comprising
- at least one bolster (11) with an upper side and an underside made of at least one plastic foam;
- at least one sensor system (20), wherein the at least one sensor system is arranged in a region of the bolster adjacent to the underside of the bolster,
**characterized in that** the at least one sensor system is enclosed by the plastic foam of the bolster, wherein the seat cushion furthermore comprises:
- at least one flame-retardant fabric covering at least one side, preferably the upper side of the at least one bolster, and
- at least one cover (13) on the upper side of the flame-retardant fabric (12),
- wherein a further sensor system is provided in the bolster (11), on the bolster (11) or on the flame-retardant fabric (12) covered by the at least one cover (13) or provided in the at least one cover (13).

2. The seat cushion according to claim 1, **characterized in that** the at least one sensor system provided in the bolster comprises sensors for pressure measurement and/or strain measurement.

3. The seat cushion according to claim 1, **characterized in that** the sensor system provided in the cover serves for measuring temperature and/or humidity.

4. The seat cushion according to any of the preceding claims, **characterized in that** the at least one sensor system comprises a flexible substrate with at least one sensor arranged on the flexible substrate.

5. The seat cushion according to any of the preceding claims, **characterized in that** the at least one sensor system is foamed or embedded into the plastic foam of the bolster.

6. The seat cushion according to any of the preceding claims, **characterized in that** the sensors of the at least one sensor system in the bolster are arranged in the buttocks area and/or along the thighs of a sitting passenger.

7. The seat cushion according to any of the preceding claims, **characterized by** at least one flame-retardant fabric comprising
- at least one barrier layer comprising at least one fiber fleece made of at least one flame-retardant type of fiber, and
- at least one abrasion-resistant layer provided on the barrier layer, comprising at least one textile fabric with high abrasion resistance made of at least one type of fiber, preferably of at least two types of fiber.

8. The seat cushion according to any of the preceding claims, **characterized in that** the at least one flame-retardant fabric includes at least one intumescence layer.

9. The seat cushion according to any of the preceding claims, **characterized in that** the at least one bolster consists of a plastic foam, preferably of polyurethane foam, polyethylene foam, polyether foam, polyester foam, silicone foam, or of a plastic fabric, preferably of polypropylene, polyethylene, polyacrylate.

10. An aircraft seat, comprising at least one seat cushion according to any of the preceding claims.

11. Use of data measured by sensor systems arranged in a seat cushion according to any of claims 1-11 in combination with a management system for monitoring the degree of wear and a predictive maintenance of the seat cushion as well as the sitting behavior of the passenger, the management system comprising
- at least one processing unit (PU) for processing the data measured by the sensor systems arranged in the seat cushion;
- at least one storage unit (SU) for storing the measured and processed data, and
- at least one display unit for displaying the processed data.

12. Use of data measured by sensor systems arranged in a seat cushion according to any of claims 1-11 in a computer-implemented method for determining the seat occupation, monitoring the degree of wear and a predictive maintenance of the seat cushion as well as the sitting behavior of the passenger, wherein the computer-implemented method comprises the following steps:
- measuring the change of at least one parameter selected from temperature, pressure and/or strain by the sensor systems arranged in the cushion according to any of claims 1-11, wherein the change in parameter is exerted / caused by a passenger sitting on the seat cushion;
- processing the measured data in the at least one processing unit and storing the data in the at least one storage unit of the management system, and
- displaying the processed data on the at least one display unit.

## Revendications

1. Coussin de siège (10) pour un siège d'avion, comprenant
- au moins un rembourrage (11) avec une surface supérieure et une surface inférieure composé d'au moins une mousse plastique ;
- au moins un système de capteurs (20), l'au moins un système de capteurs étant agencé dans une zone du rembourrage adjacente à la surface inférieure du rembourrage,
**caractérisé en ce que** l'au moins un système de capteurs est enfermé par la mousse plastique du rembourrage, le coussin de siège comprenant en outre :
- au moins un tissu ignifuge (12) couvrant au moins un côté, de préférence la surface supérieure de l'au moins un rembourrage, et
- au moins un revêtement (13) sur la surface supérieure du tissu ignifuge (12),
- où un autre système de capteurs dans le rembourrage (11), sur le rembourrage (11) ou sur le tissu ignifuge (12) est prévu couvert de l'au moins un revêtement (13) ou est prévu dans l'au moins un revêtement (13).

2. Coussin de siège selon la revendication 1, **caractérisé en ce que** l'au moins un système de capteurs prévu dans le rembourrage comprend des capteurs pour la mesure de la pression et/ou la mesure de la contrainte.

3. Coussin de siège selon la revendication 1, **caractérisé en ce que** le système de capteurs prévu dans le revêtement sert à mesurer la température et/ou l'humidité.

4. Coussin de siège selon l'une quelconque des revendications précédentes, **caractérisé en ce que** l'au moins un système de capteurs comprend un substrat flexible avec au moins un capteur agencé sur le substrat flexible.

5. Coussin de siège selon l'une quelconque des revendications précédentes, **caractérisé en ce que** l'au moins un système de capteurs est moulé ou intégré dans la mousse plastique du rembourrage.

6. Coussin de siège selon l'une quelconque des revendications précédentes, **caractérisé en ce que** les capteurs de l'au moins un système de capteurs sont agencés dans le rembourrage dans la région du postérieur et/ou le long des cuisses d'un passager assis.

7. Coussin de siège selon l'une quelconque des revendications précédentes, **caractérisé par** au moins un tissu ignifuge, comprenant
- au moins une couche barrière, comprenant au moins un tissu non-tissé composé d'au moins un type de fibre résistant aux flammes, et
- au moins une couche résistante à l'abrasion prévue sur la couche barrière comprenant au moins un matériau textile à haute résistance à l'abrasion d'au moins un type de fibre, de préférence au moins deux types de fibres.

8. Coussin de siège selon l'une quelconque des revendications précédentes, **caractérisé en ce que** l'au moins un tissu ignifuge présente au moins une couche d'intumescence.

9. Coussin de siège selon l'une quelconque des revendications précédentes, **caractérisé en ce que** l'au moins un rembourrage est composé d'une mousse plastique, de préférence de mousse de polyuréthane, de mousse de polyéthylène, de mousse de polyéther, de mousse de polyester, de mousse de silicone ou d'un tissu plastique, de préférence de polypropylène, de polyéthylène, de polyacrylate.

10. Siège d'avion, comprenant au moins un coussin de siège selon l'une quelconque des revendications précédentes.

11. Utilisation de données mesurées par des systèmes de capteurs agencés dans un coussin de siège selon l'une quelconque des revendications 1-11 en combinaison avec un système de gestion pour surveiller le degré d'usure et une maintenance prédictive du coussin de siège ainsi que le comportement du passager assis, le système de gestion comprenant
- au moins une unité de traitement pour le traitement des données mesurées par les systèmes de capteurs agencés dans le coussin de siège ;
- au moins une unité de stockage pour stocker les données mesurées et traitées, et
- au moins une unité d'affichage pour afficher les données traitées.

12. Utilisation de données mesurées par des systèmes de capteurs agencés dans un coussin de siège selon l'une quelconque des revendications 1-11 dans un procédé mis en œuvre par ordinateur pour déterminer l'occupation des sièges, pour surveiller le degré d'usure et pour une maintenance prédictive du coussin de siège ainsi que le comportement du passager assis, ledit procédé mis en œuvre par ordinateur comprenant les étapes suivantes :
- mesurer la modification d'au moins un paramètre sélectionné parmi la température, la pression et/ou l'allongement par les systèmes de capteurs agencés dans le coussin selon l'une quelconque des revendications 1-11, la modification de paramètre étant exercée / provoquée par un passager assis sur le coussin de siège ;
- traiter les données mesurées dans l'au moins une unité de traitement et stocker les données dans l'au moins une unité de stockage du système de gestion, et
- afficher les données traitées sur l'au moins une unité d'affichage.
